(19)

Europäisches
Patentamt
European
Patent Office
Office européen
des brevets

(11) **EP 2 412 435 A2**

(12) **EUROPEAN PATENT APPLICATION**

(43) Date of publication:
**01.02.2012 Bulletin 2012/05**

(51) Int Cl.:
***B01J 20/32*** (2006.01)   ***B01J 20/286*** (2006.01)
***B01J 39/26*** (2006.01)   ***B01J 41/20*** (2006.01)

(21) Application number: **11173861.3**

(22) Date of filing: **13.07.2011**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **28.07.2010 US 368386 P**

(71) Applicant: **Rohm and Haas Company
Philadelphia,
Pennsylvania 19106-2399 (US)**

(72) Inventors:
• **Deetz, Martin J.
North Wales, PA Pennsylvania 19454 (US)**
• **Maikner, John J.
Old Zionsville, PA Pennsylvania 18068 (US)**

(74) Representative: **Buckley, Guy Julian
Patent Outsourcing Limited
1 King Street
Bakewell
Derbyshire DE45 1DZ (GB)**

(54) **Process for making improved chromatography media and method of use**

(57)    The present invention further is directed to the improved process for making by free radical polymerization a monodisperse, macroporous polymeric media with covalently bonded polymer chains. The media can be applied for chromatographic purification, resulting in polymer supports having improved protein binding capacity and resin selectivity, as well as methods relating to making and using the same.

EP 2 412 435 A2

**Description**

The present invention is directed to an improved process for making by free radical polymerization a monodisperse, macroporous polymeric media with covalently bonded polymer ligand chains. The media can be applied for chromatographic purification, resulting in polymer supports having improved protein binding capacity, kinetics and selectivity, as well as methods relating to making and using the same.

Therapeutic proteins produced from living organisms play an increasingly important role in modern healthcare. These proteins provide many advantages over traditional pharmaceuticals, including increased specificity and efficacy towards disease targets. Mammalian immune systems use a range of proteins to control and eliminate disease threats. The advent of genetic and protein engineering has allowed the development of many "designed" or recombinant protein therapeutics. These therapeutics can be based on a single protein, chemically modified protein, protein fragment or protein conjugate. Chromatographic separations are extensively utilized in the manufacturing of these biopharmaceuticals. As the industry matures, implementation of novel/advanced technologies and methods to enhance separations will provide biotherapeutic producers the ability to provide these medicines to more patients and at lower cost.

Common chromatography methods used to purify proteins include affinity, bioaffinity, ion exchange, reversed phase, hydrophobic interaction, hydrophilic interaction, size exclusion and mixed mode (resin containing combinations of the aforementioned categories) among others. The application and efficiency of each of those types of chromatography procedures relies on the selectivity of surface-surface interactions between the solute molecules and the stationary phase of the chromatography system (chromatography media), each interacting with the mobile liquid phase. A wide variety of stationary phase chromatography support materials are commercially available.

Often the key to a successful separation of product from impurities relies on the correct combination of stationary phase, base matrix (chemical composition and pore structure) and ligand properties (ligand type, ligand density, pore structure, ligand distribution, ligand length and material composition), and mobile phase or solution properties (buffer type, pH and conductivity). The specific design of the base matrix and ligand results in a chromatography media which can be characterized by several key attributes including protein binding capacity, process throughput, selectivity, bed permeability and chemical stability. Purification methods include predominately binding the product (bind and elute), predominately binding the impurities (flow-through) and combinations of the aforementioned (so called weak partitioning and others). It is critical in the design of these technologies to control the chromatography media properties taught above in order to enable and ensure a robust separation leading to purified protein product.

Protein separations can be accomplished on a variety of polymer supports or base matrices. Common materials for resin or bead structures include polysaccharides (agarose, cellulose), synthetic polymers (polystyrene, polymethacrylate, polyvinylethers and polyacrylamide) and ceramics such as silica, zirconia and controlled pore glass. These materials adsorb proteins predominately via "diffusive pores" which are typically about 200 Å to 3,000 Å.

Membrane and monolith materials are also commonly used for chromatography, particularly in flow-through applications. Typical membrane compositions include synthetic polymers such as polyvinylidenefluoride, polyethylene, polyethersulfone, nylon, and polysaccharides such as cellulose. Monoliths have been developed from polystyrene, polysaccharides, polymethacrylate and other synthetic polymers, polysaccharides and ceramics. Membrane and monolith chromatography differs from beads in that these materials adsorb proteins in the same "convective pores" which control the membrane and monolith material's permeability. Typical membrane and monolith convective pore sizes range from about 0.6μm to about 10μm. Ligand addition to these substrates can be accomplished through a variety of well developed techniques.

The use of polymeric ligand "tentacles" or "extenders" to improve protein binding capacity and modify resin selectivity involves coupling a polymeric ligand onto a base matrix such as by grafting, which extend away from the base matrix surface. Polymeric ligand extenders typically create greater binding capacity and kinetics because the extenders increase ligand availability because target molecule binding to the polymeric ligands exceeds that of a monolayer adsorption of protein on the polymer support surface.

Two standard methodologies for grafting polymer extenders have been developed for creating surface extenders on polymer supports such as those used in chromatography for protein separation and the like: 1) grafting of monomers from a support via a surface radical ("grafting monomers from"), and 2) grafting a preformed polymer to a support via an activating group ("grafting polymers to").

Grafting monomers from polymeric materials using radical polymerization reactions is a well developed technology. In general, the reaction can be initiated from a polymeric surface material, or from an initiator in solution.

Initiating the radical polymerization from the surface can be accomplished by generating radicals at the surface via exposure to reactive environments such as temperature, radiation, metal oxidation or initiation through adsorbed initiating species. However, these "grafting monomers from" approaches require very controlled solution conditions special equipment and/or require that the support be pre-activated in some way such as by pretreatment with a polymerizable linker, initiating species or specific functional groups which make their implementation complicated and time consuming.

One method for grafting polymers from porous materials is disclosed in US5453186. The art discloses a method of grafting polymer ligands onto a polymer support having dihydroxyl functionality. The art discloses a method controlling the chain length and grafting to a support containing hydroxyl groups by the use of redox polymerization. The Ce(IV) redox method employs use of a cerium(IV) salt under inert atmosphere to graft polymeric ligands from the polymer support surface. The polymer ligand chain length is controlled by controlling the level of monomers and cerium salt concentration added to the mixture.

Although the art discloses a method for grafting polymers from polymeric materials, it requires the pretreatment of the polymer support with specific functional groups. A method is needed where this additional pretreatment step can be removed from the process while maintaining resultant performance of the overall polymeric chromatographic material.

The addition of polymeric ligands to a polymeric support material surface provides improved protein binding capacity, protein binding kinetics and potential changes in protein selectivity. However, as protein separations become more demanding, it becomes more critical to develop new technologies and methods in order to create novel polymeric structures. Accordingly, it would be desirable to develop improved protein binding properties and protein selectivity of a wider range of polymeric support substrates used in protein separation.

In response to the above needs for new polymeric substrates, useful for protein separations, having improved protein binding capacity and resin selectivity, a new method for grafting polymeric ligands onto polymeric support substrates has been developed.

According to the present invention there is provided an adsorbent material for chromatography comprising a polymeric ligand immobilized onto a polymeric support,

wherein the polymeric support comprises suitable free radical grafting sites,

further wherein the polymeric ligand is attached to the suitable free radical grafting sites, of the polymer support by one or more covalent bonds.

In a second aspect of the invention there is provided a method of preparing an adsorbent material for chromatography comprising:

(i) providing a polymeric support comprising suitable free radical grafting sites
(ii) generating a polymeric ligand by the polymerization of functional monomers; and
(iii) initiating a reaction between the polymeric support and the polymeric ligand
wherein the polymeric ligand reacts with the suitable free radical grafting sites on the polymeric support.

In a third aspect of the present invention there is provided a method of separating chemical compounds from a mixture comprising

(i) providing a mixture and
(ii) contacting the mixture with an adsorbent material for chromatography comprising a polymeric ligand immobilized onto a polymeric support,
wherein the polymeric support comprises suitable free radical grafting sites,
further wherein the polymer ligand is attached to suitable free radical grafting sites,
of the polymer support by one or more covalent bonds.

All ranges taught herein are to be understood to encompass all subranges subsumed therein. For example, a range of "1 to 10" includes any and all subranges between (and including) the minimum value of 1 and the maximum value of 10, that is, any and all subranges having a minimum value of equal to or greater than I and a maximum value of equal to or less than 10, e.g., 5.5 to 10.

As used herein, the singular forms "a," "an," and "the" include plural referents unless the context clearly dictates otherwise.

As used herein, the terms "polymer ligand" and "polymeric ligand" are synonymous terms as are the terms "polymeric support" and "polymer support."

As used herein, the terms "preferably," "more preferably," and "most preferably" are not intended to suggest a mode that is deemed to be superior but instead is meant to embody suitable narrower ranges of a broader range or group.

The present invention does not require polymeric support pretreatment or the addition of a polymerizable linker to the polymeric support as compared to the processes of the prior art. The polymeric ligand that is generated by the polymerization of functional monomers and/or chain transfer reagents and initiators will react with the suitable free radical grafting sites on the polymeric support and/or polymeric support backbone and become covalently attached. The initiator will initiate polymerization of the monomer in solution which will graft onto the surface at the suitable free radical grafting sites of the polymeric support.

The polymeric support of the present invention includes any polymeric material which has a suitable surface to graft polymeric ligands having a free radical species. Although a polymerizable linker is not required by the present invention one may be present in the system without any disruptive effect. The polymeric supports of the present invention may be prepared by suspension, emulsion or dispersion polymerization or combinations thereof.

The polymeric support material can be a particulate material, or, in the form of a piece, a sheet, tube, a rod, or a capillary coating. Preferably, the support material is a particulate material, suitably having a volume average particle size of from about 0.5 to about 1500 $\mu$m, preferably from about 0.7 to about 800 $\mu$m, most preferably from about 10 to about 150 $\mu$m. The particles are preferably substantially spherical. The particulate material suitably comprises pores. The pore size, pore volume and specific surface area of the particulate support material may vary depending on the type of support material used, the characteristics of the polymer to be linked to the support and the desired separation characteristics when in use. The pore size range of particulate particles may be from about 20 to about 4000 Å, preferably from about 200 to about 4000 Å, most preferably from about 300 to about 1500 Å The pore volume is suitably from about 0. 1 to about 4 ml/g, preferably from about 0.3 to about 2 ml/g, most preferably from about 0.5 to about 1.5 ml/g. The specific surface area is suitably from about I to about 1000 m$^2$/g, preferably from about 25 to about 700 m$^2$/g. most preferably from about 50 to about 500 m$^2$/g.

Other "polymeric supports" or "base matrices" that can be used herein include, but are not limited to, any material with diffusive pores. Preferred materials for polymer supports or base matrices that can be used herein include polysaccharides, synthetic polymer, agarose, cellulose, polymethacrylates, polyacrylates, polyacrylamides, polymethacrylamides, polystyrene, polyvinylether, and hybrids or combinations of the aforementioned. The polymeric supports of the present invention have suitable free radical grafting sites which enable the polymeric ligand to bond by covalent bonds.

The temperature during the free radical polymeric ligand grafting reaction is suitably from about 0 to about 100° C, preferably from 15 to 50° C and alternatively from 50 to 90° C. Suitably, the reaction mixture is kept under an air atmosphere but could be performed under an inert atmosphere.

In some embodiments, the polymeric ligands may contain functional groups. Examples of "functional groups" that can be used herein include, but are not limited to, ion exchange groups, bioaffinity groups, hydrophobic groups, thiophilic interaction groups, chelate or chelating groups, groups having so called pi-pi interactions with target compounds, hydrogen bonding groups, hydrophilic groups and unfunctional monomers or intermediary monomers capable of further transformation into another functional group (e.g. glycidyl methacrylate which is transformed into an ion exchange ligand), and mixtures thereof..

Examples of "polymeric ligands" that can be used herein include, but are not limited to, polymers containing ion exchange groups, hydrophobic interaction groups, hydrophilic interaction groups, thiophilic interactions groups, metal affinity groups, affinity groups, bioaffinity groups, mixed mode groups and unfunctional polymers or intermediary polymers capable of further transformation into another functional group (e.g. poly glycidyl methacrylate which is transformed into an ion exchange polymeric ligand), and mixtures thereof. Some preferred ligands that can be used herein include, but are not limited to polymers containing, strong cation exchange groups, such as sulphopropyl, sulfonic acid; strong anion exchange groups, such as trimethylammonium chloride; weak cation exchange groups, such as carboxylic acid; weak anion exchange groups, such as N,N-diethylaminoethyl.

In some embodiments of this chemistry, it may be relevant to add chain transfer reagents to the reaction medium when the polymeric ligands are generated. Suitable chain transfer reagents include, for example, halomethanes, disulfides, thiols (also called mercaptans), and metal complexes. Additional suitable chain transfer reagents include various other compounds that have at least one readily abstractable hydrogen atom, and mixtures thereof. Chain transfer reagents may be added in one or more additions or continuously, linearly or not, over most or all of the entire reaction period or during limited portions of the reaction period. The chain transfer reagents can be added to the reaction at levels from 0.01 % to 15%, more preferably from 0.1 % to 3 %, most preferably from about 0.1 % to 1 %.

Examples of "free radical initiators" that can be used herein include, but are not limited to any free radical initiator capable of reacting with vinyl monomers to form polymeric ligands such as peroxides, such as tert-butylhydroperoxide, cumene hydroperoxide; peroxyacetates, such as peracetic acid, chloroperbenzoic acid; persulfates, such as ammonium persulfate, sodium persulfate, potassium peroxodisulfate, azo initiators such as 4,4'-azobis(4-cyanovaleric acid), Irgacure® 2959 (Ciba-Geigy, Hawthorn, N.Y.), 2,2'-azobis(2-amidino-propane)hydrochloride, and the like and mixtures thereof. The grafting to reaction can be initiated with methods known in the art, preferably thermal initiation (heating) or UV irradiation. The free radical initiators can be water soluble or oil soluble. The initiators are added to the reaction at levels from 0.01% to 5%, more preferably from 0.1 % to 2%.

Examples of "functional monomers" or "mixture of grafting monomers" that can be used to prepare the polymeric ligands herein include, but are not limited to, methacrylates, acrylates, methacrylamides and acrylamides. The functional monomers include, but are not limited to, acrylic acid, 2-acrylamido-2-methyl-1-propanesulfonic acid, [3-(methacryloylamino) propyl] trimethylammonium chloride, 2-acrylamido-glycolic acid, itaconic acid or ethyl vinyl ketone, glycidyl methacrylate, N,N-dimethylacrylamide, acrylamide, hydroxypropyl methacrylate, N-phenylacrylamide, hydroxyethyl acrylamide, and combinations thereof.

In the grafting method taught herein, the polymerization of the polymer ligand is initiated in solution under less restrictive conditions than growth of the polymeric ligand from the surface of the polymer support.

Crosslinkers, branching agents and nonfunctional monomers may be attached on the polymeric ligand for the purpose of controlling the morphology or interaction of the polymeric ligands. However, these crosslinkers or branching agents on the polymeric ligand are present at low levels, suitable from <5%, more preferably <1%. Suitable crosslinkers or branching agents include but are not limited to monomers, such as ethylene glycol dimethacrylate, divinyl benzene, trimethylpropyl trimethacrylate and methylene bisacrylamide or multifunctional chain transfer reagents

The present invention further comprises the use of the adsorbent material for chromatography in chromatographic separation methods. Such chromatographic separation methods can be, for example, low pressure liquid chromatography, medium pressure liquid chromatography, HPLC, supercritical fluid chromatography (SFC), and simulating moving bed (SMB).

The present invention further comprises a method of separating chemical compounds from a mixture comprising contacting the mixture with the adsorbent material for chromatography according to the present invention.

The primary advantage of the invention is the generation of high capacity ion exchange resins using the ligand grafting for chromatographic capture and or purification of molecules, and more specifically, biomolecutes

TEST METHODS

Determination of the Uptake Binding Capacity:

Preparation of Solution 1 (50mM Tris/HCl, pH 8.8)

12.1 g of Tris(hydroxymethyl) aminomethane (commercially available from Fisher Scientific) was added to a 2 L volumetric flask. 0.01N HCl solution (commercially available from Fisher Scientific) was applied to fill the flask to 2 liter mark. The contents were shaken after the volumetric flask was capped. The solution rested for 5 min and the volume of solution was rechecked. Additional HCl was added to adjust the volume to 2 L mark. The pH was measured at $8.8 \pm 0.05$. The solution was labeled and refrigerated at 4 °C.

Preparation of Solution 2: 2 mg/ml BSA (albumin from bovine serum, (commercially available from Sigma-Aldrich) solution

0.806 g of BSA was weighed in a glass jar and added into 403 g of Solution 1. The contents were mixed gently to dissolve. The solution sat for 0.5 hour to ensure full BSA dissolution.

BSA capacity uptake procedure

1 ml of DI water was slowly added to one Bio-Rad Poly-Prep Chromatography Column to prevent any trapped air. The height of the water was marked. The end-plug was removed from the column. The column was connected to a manifold in a ventilation hood. More DI water was added to the column, and gentle vacuum was applied to the column until water level was 1-2 cm to remain in the bottom of the column to prevent air entrapment. The slurry solution of Example 7 was added into the column. A head of water above the resin was always kept. 1 ml of resin in the column was measured. Gentle vacuum was applied to remove all but 1-2 cm of solution above the resin. When the resin level fell below the 1ml mark, more resin slurry was added with additional vacuum. The resin bead was never exposed to air. The packed 1 ml resin was rinsed with approximately 10 ml of DI water to displace the slurry solution and the liquid was allowed to drain until the water level is 1-2 cm above the packed bed. Then the packed 1 ml resin were flushed with 10 ml of Solution 1 from Example 6. Solution 1 was removed from the column by vacuum and air was pulled through the column for 1 minute. The disposable column containing the wet cake was removed from the manifold.

The wet cake from the column was transferred to an 8oz glass jar with a spatula and 200 mL of solution 2 (2 mg/mL BSA sln.) was added. The sample in the glass jar was gently shaken for 18h.

After 18 hours, the sample was removed from the shaker and the resin was allowed to settle for 15 minutes.

The BSA binding capacity was determined from the 278 nm UV absorbance of the filtered supernatant BSA solution after 18 hours incubation. A cuvette filled with solution 1 was used to zero the UV spectrometer. The absorbance at 278 nm was measured for all the samples, standards solutions, and control sample (Q Sepharose™ Fast Flow Ion Exchange Resin, commercially available from GE Healthcare).

Calculation of the BSA Binding Capacity:

The values for unbound BSA for both the sample and control were recorded (Q Sepharose FF).

The following equation was applied to determine the binding capacity:

Equation 1

$$\text{BSA capacity} = [400-(\text{sample mg/mL} \times 200)] \times 1\ \text{mL} = \text{mg of BSA/mL of resin}$$

EXAMPLE 1

Preparation of 60 um GMA/GlyDMA porous Anion Exchanger

In a 250 ml glass reactor equipped an overhead stirrer, condenser and temperature probe, was charged 50 g of a monosized GMA/GlyDMA (60 $\mu$m polyGMA-GlyDMA beads glycidyl methacrylate (GMA) and glycerol-1,3-dimethacrylate (GlyDMA), produced by a process disclosed in US patent application 1/521,668, Example 25: Preparation 60 micrometer GMA/EGDMA polymeric resin ), copolymer wet cake (~25 % solids), to the wet cake a 18 grams charge of 75 % (3-acrylamidopropyl)-trimethylammonium chloride ( commercially available at Sigma- Aldrich Chemical company) added to the reactor. Then 0.6 grams of ammonium persulfate (commercially available at Acros organics-Fisher Scientific), 0.6 2,2 Azobis (2 methylpropion amidine) dihydrochloride (commercially available at Sigma- Aldrich Chemical company) and 68 grams of D.I. water was added to the reactor.

The temperature of the reactor was maintained at 25 °C for 30 minutes with stirring. The reactor was heated to 80 °C over 45 minutes in air. Reactor maintained a temperature of 80 °C for 5 hour in air and cool to 50 °C. Over 1 hour 120 mls of D.I. water was added using a 60 mls addition funnel and maintaining a constant temperature of 50 °C with stirring. The reaction mixture was filtered using 500 mls fritted buchner funnel and to wash the bead of the polymer solution. One liter of D.I. water was used. The Bovine serum albumin (BSA) equilibrium binding capacity measured on the resin. BSA Capacity 141 mg/ml.

EXAMPLE 2

Preparation of 60 um GMA/GlyDMA porous Anion Exchanger with N,N,N,N Tetramethyl ethylenediamine

In a 250 ml glass reactor equipped an overhead stirrer, condenser and temperature probe, was charged 50 g of a monosized GMA/GlyMA copolymer wet cake (~25 % solids), to the wet cake a 30 g charge of 75 % (3-acrylamidopropyl) Trimethylammonium chloride (commercially available at Sigma- Aldrich Chemical company) added to the reactor. Then 0.6 grams of ammonium persulfate (commercially available at Acros Organics --Fisher Scientific), 0.6 2,2 Azobis (2 methylpropion amidine) dihydrochloride (commercially available at Sigma- Aldrich Chemical company), 20 uls of N, N,N,N Tetramethyl ethylenediamine (commercially available at Sigma-Aldrich Chemical company) and 68 grams of D.I. water was added to the reactor.

The temperature of the reactor was maintained at 25 °C for 30 minutes with stirring, then the reactor was heated to 80 °C over 45 minutes in air. Reactor maintained a temperature of 80 °C for 5 hour in air and cool to 50 °C. Over 1 hour 120 mls of D.I. water was added using a 60 mls addition funnel and maintaining a constant temperature of 50 °C with stirring. The reaction mixture was filtered using 500 mls fritted buchner funnel and to wash the bead free the polymer solution. One liter of D.I. water was used to wash the beads. The BSA (commercially available at albumin from bovine serum, Sigma-Aldrich)batch equilibrium binding capacity measured on the resin. Bovine serum albumin BSA Capacity 188 mg/ml.

EXAMPLE 3

Preparation of 60 um GMA/GlyDMA porous Anion Exchanger via a hydrolyzed copolymer

In a 250 ml glass reactor equipped an overhead stirrer, condenser and temperature probe, was charged 50 g of a monosized GMA/GlyMA copolymer wet cake (~25 % solids), to reactor 150 mls 1 M Sodium Hydroxide solution (commercially available at Fisher Scientific company) was added. The mixture stirring for 48 hour at 25 °C and the reaction mixture was filtered using 500 mls fritted buchner funnel and to wash the bead free sodium hydroxide solution. One liter of D.I. water was used to wash the beads.

Then 30 g of this wet cake materials was charged back into 250 ml glass reactor, 18 g charge of 75 % (3-acrylamidopropyl) Trimethylammonium chloride (commercially available at Aldrich Chemical company) added to the reactor. Then 0.36 g of ammonium persulfate (commercially available at Acros organics-Fisher Scientific company, 40.5 grams of D.I. water was added to the reactor.

The temperature of the reactor was maintained at 25 °C for 30 minutes with stirring. The reactor was heated to 80 °C over 45 minutes in air. Reactor maintained a temperature of 80 °C for 18 hour in air and cool to 50 °C. Over 1 hour 120 mls of D.I. water was added using a 60 mls addition funnel and maintaining a constant temperature of 50 °C with stirring. The reaction mixture was filtered using 500 mls fritted buchner funnels and to wash the bead free of the polymer solution. One liter of D.I. water was used. The BSA(albumin from bovine serum, commercially available at Sigma-Aldrich) batch equilibrium binding capacity measured on the resin. Bovine serum albumin BSA Capacity 101 mg/ml.

Example 4

Preparation of 60 μm GMA/GlyDMA porous weak base Anion Exchanger

In 250 ml glass reactor equipped an overhead stirrer, condenser and temperature probe, was charged 30 g of monosized GMA/GlyDMA copolymer wet cake (~25 % solids).
Charged 18 grams of N-[3-(Dimethylamino)prohyl]methacrylamide (commercially available at Aldrich Chemical company), 0.36 grams of ammonium persulfate (commercially available at Acros Organics-Fisher Scientific company) and 40.5 grams of D.I. water to the reactor
Let the reactor stir for 30 min at room temperature, then heated the reactor to 80°C over 45 minutes. Maintained the reactor temperature at 80°C for 22 hour in air, then cooled the reactor to 50°. By funnel added 60 ml of D.I. water over 30 minutes with stirring and maintained a constant temperature at 50°C.
Cooled reaction mixture to room temperature. Then transferred the reaction mixture to 600 ml fritted buchner funnel and filtered the bead free of the polymer solution. Washed beads with one liter of D.I. water over 1 hour. BSA capacity (Bovine serum albumin) 110 mg/ml

Example 5

Preparation of 60 um GMA/GlyDMA porous Anion Exchanger with Chain Transfer Reagent

In a 2000 ml glass reactor equipped an overhead stirrer, condenser and temperature probe, was charged 300 g of a monosized GMA/GlyMA copolymer wet cake (~25 % solids), to the wet cake a 180 g charge of 75 % (3-acrylamidopropyl) Trimethylammonium chloride (Sigma-Aldrich Chemical company) added to the reactor. Then 0.9 grams of ammonium persulfate (Acros Organics-Fisher Scientific company), 60 mg of Butyl 3-mercapto propionate Sigma-Aldrich Chemical company) and 405 grams of D.I. water was added to the reactor.
The temperature of the reactor was maintained at 25 °C for 30 minutes with stirring The reactor was heated to 80 °C over 45 minutes in air. Reactor maintained a temperature of 80 °C for 22 hour in air and cool to 50 °C. Over 1 hour 500 mls of D.I water was added using a 600 mls addition funnel and maintaining a constant temperature of 50 °C with stirring. The reaction mixture was filtered using 2000 mls fritted buchner funnels and to wash the bead free of the polymer solution. Ten liter of D.I. water was used. The BSA(albumin from bovine serum, Sigma-Aldrich) batch 171 mg/ml.

**Claims**

1. An adsorbent material for chromatography comprising:

   a polymeric ligand immobilized onto a polymeric support,
   wherein the polymeric support comprises suitable free radical grafting sites,
   further wherein the polymeric ligand is attached to the suitable free radical grafting sites of the polymer support by one or more covalent bonds.

2. The adsorbent material for chromatography of claim 1 wherein the polymeric ligands comprise a free radical species.

3. The adsorbent material for chromatography according to claim 1, wherein the polymeric ligand comprises functional groups.

4. The adsorbent material for chromatography according to claim 1 wherein the polymeric ligands comprise ion exchange groups, hydrophobic interaction groups, hydrophilic interaction groups, thiophilic interactions groups, metal affinity groups, affinity groups, bioaffinity groups, mixed mode groups and unfunctional monomers or intermediary monomers capable of further transformation into another functional group or mixtures thereof.

5. The adsorbent material for chromatography according to claim, wherein the polymeric support has a volume average particle size of from .5 to about 500 $\mu$m.

6. A method of preparing an adsorbent material for chromatography comprising:

(i) providing a polymeric support comprising suitable free radical grafting sites
(ii) generating a polymeric ligand by the polymerization of functional monomers; and
(iii) initiating a reaction between the polymeric support and the polymeric ligand
wherein the polymeric ligand reacts with the suitable free radical grafting sites on the polymeric support.

7. The method of preparing an adsorbent material for chromatography of claim 6 wherein a free radical initiator is added to initiate a reaction between the polymeric support and the polymeric ligand.

8. The method of preparing an adsorbent material for chromatography of claim 7 wherein the free radical initiator is a peroxide, peroxyacetate, persulfate, azo initiator, or mixtures thereof.

9. The method of preparing an adsorbent material for chromatography of claim 6, wherein the polymeric support has a volume average particle size of from .5 to about 500 $\mu$m.

10. The method of preparing an adsorbent material for chromatography of claim 6 wherein the generating a polymeric ligand by the polymerization of functional monomers is in the presence of chain transfer reagents.

11. A method of separating chemical compounds from a mixture comprising

(i) providing a mixture and
(ii) contacting the mixture with an adsorbent material for chromatography comprising a polymer ligand immobilized onto a polymeric support,
wherein the polymeric support comprises suitable free radical grafting sites,
further wherein the polymer ligand is attached to suitable free radical grafting sites, of the polymer support by one or more covalent bonds.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5453186 A
- US 1521668 A